# EUROPEAN PATENT APPLICATION

(11) **EP 2 468 177 A1**
(43) Date of publication of application: **27.06.2012**
(21) Application number: 11194837.8
(22) Date of filing: 21.12.2011
(51) Int. Cl.: A61B 5/00

(54) **Delineating skin or surface lesions**

(30) Priority: 23.12.2010 US 977372
(71) Applicant: Tyco Healthcare Group, LP, Mansfield, MA 02048 (US)
(72) Inventor: Keller, Craig A., Boulder, CO Colorado 80303 (US)
(74) Representative: HOFFMANN EITLE

(57) **Abstract**

A diagnostic apparatus for examining a region of skin includes a light source for emitting light toward an illumination region of the skin surface. A detector is provided for receiving light from an examination region of the skin surface. A light barrier substantially impervious to the light emitted from the light source intimately engages the skin surface to define a boundary between the illumination region and the examination region.

## Description

### BACKGROUND

### 1. Technical Field

The present disclosure relates generally to detecting and characterizing surface or skin features. In particular, the disclosure relates to an apparatus for identifying and delineating lesions in the skin by analyzing light passing through the skin.

### 2. Background of Related Art

Early and accurate detection of skin lesions such as melanoma is crucial to providing a patient with a successful treatment. Traditionally, performing a biopsy on each suspected lesion was the most effective diagnostic tool available to healthcare providers. However, since biopsies require removal of tissue that can be painful to a patient, particularly when the patient is subject to a large number of biopsies, less invasive methods may be preferred.

Optical methods have now been developed for identifying and characterizing a suspected skin lesion in which light reflected from a suspected lesion and the surrounding skin is analyzed. These methods typically involve directing light with known parameters toward the skin in which the lesion is suspected, and monitoring the reflection, absorption and refraction of that known light. The spatial distribution of a reflective signal may be analyzed with respect to intensity, wavelength, color or other optical characteristics to identify the boundary between normal and anomalous skin. Identifying the boundary of lesions having microscopic dimensions can be instrumental in an early cancer diagnosis. In other instances, measurements of the intensity of light reflected in certain frequency bands may reveal characteristics of the suspected lesion. For example, some anomalous skin conditions are known to have distinguishing signatures in the near infrared frequency bands. Data describing these signatures may be stored in the memory of an analyzer or computer for comparison with data measured during the optical examination of a suspected lesion.

In some instances these optical methods may be more effective when only light passing through the skin is analyzed. For instance, for a subsurface lesion, light reflected from the surface of the skin may not reveal information as valuable as light penetrating the skin and actually encountering the lesion. One method employed to analyze only the light passing through the skin is to detect light passing through an entire body part rather than the light reflected. While this method may be effective for some narrow body parts, such as the hand, it may prove difficult for more substantial areas. Accordingly, a diagnostic apparatus for analyzing reflected light known to pass through the skin would be helpful.

### SUMMARY

The present disclosure describes an apparatus for interrogating an area of skin. The diagnostic apparatus includes a light source for emitting light toward an illumination region of the skin surface, a detector for receiving light from an examination region of the skin surface, and a light barrier substantially impervious to the light emitted from the light source. The light barrier intimately engages the skin surface to define a boundary between the illumination region and the examination region and to discourage or prevent light emitted from the light source not passing through the skin from entering the examination region.

The light source may be configured to emit light in the visible and near infrared frequency bands. Also, the light source may be configured to vary the frequency of the emitted light.

The detector may be configured to spatially resolve light received from the examination region.

The illumination region may be enclosed between first and second walls. The first and second walls may be formed in a closed shape with respect to enclosing the illumination and examination areas of the skin in the light barrier.

The first wall and/or the second wall may be arranged to be brought into intimate contact with the skin.

In an embodiment, the light barrier is configured so that only light passing through the examination region of the skin is received by the detector.

The light barrier may include a first annular or other closed shaped wall substantially encircling or enclosing the examination region. The illumination region may substantially encircle or otherwise surround the first wall, and the light source may be configured to emit light substantially evenly about the illumination region. The light barrier may include a second annular or other closed shaped wall substantially encircling or enclosing the illumination region.

The light source and the detector may be fixedly coupled to the light barrier such that a spatial relationship is maintained therebetween.

The detector may include a camera, and the camera may be configured to digitize an image of the examination region and transmit the digitized image to a processor for analysis. The processor may include a personal computer, and the personal computer may include at least one reference image for comparison with the digitized image of the examination region.

The detector may be optically coupled to a light transmission structure extending in the direction of the examination region and configured to guide light to the detector in a predetermined direction. The light transmission structure may include a light pipe or a fiber-optic bundle. The light transmission structure may comprise a plurality of fibre optics spread over the examination region.

According to another aspect of the disclosure, an apparatus for examining a region of skin includes a light barrier for prohibiting the passage of light therethrough in the form of an annulus. The annulus is configured for intimately contacting a skin surface to define an interior examination region substantially encircled by the annulus and an exterior illumination region substantially encircling the annulus. A light source is disposed in the exterior illumination region and is configured to emit light toward the skin surface. A detector is disposed within the interior examination region and is configured to receive light passing through the skin beneath the light barrier.

The detector may be configured to digitize an image of the examination region and transmit the digitized image to a processor for analysis. The processor may be configured to compare the digitized image to a reference image, or the processor may be configured to execute a mathematical algorithm for distinguishing malignant lesions from benign lesions. The processor may also be configured to analyze the spatial or spectral distribution of light from the examination region to identify a boundary of a suspected lesion within the skin.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1 is a schematic view of a diagnostic apparatus including a light barrier for separating an illumination region from an examination region for analyzing light in accordance with the present disclosure;
FIG. 2A is schematic view of an alternate embodiment of a diagnostic apparatus including a light barrier in the form of an annulus;
FIG. 2B is a bottom view of the light barrier depicted in FIG. 2A;
FIG. 3 is a schematic view of the diagnostic apparatus depicted in Figure 2A equipped with a light transmission structure; and
FIG. 4 is a schematic view of an alternate embodiment of a diagnostic apparatus including a first light barrier for separating an illumination region from an examination region and a second light barrier for separating the examination region from an ambient region.

### DETAILED DESCRIPTION

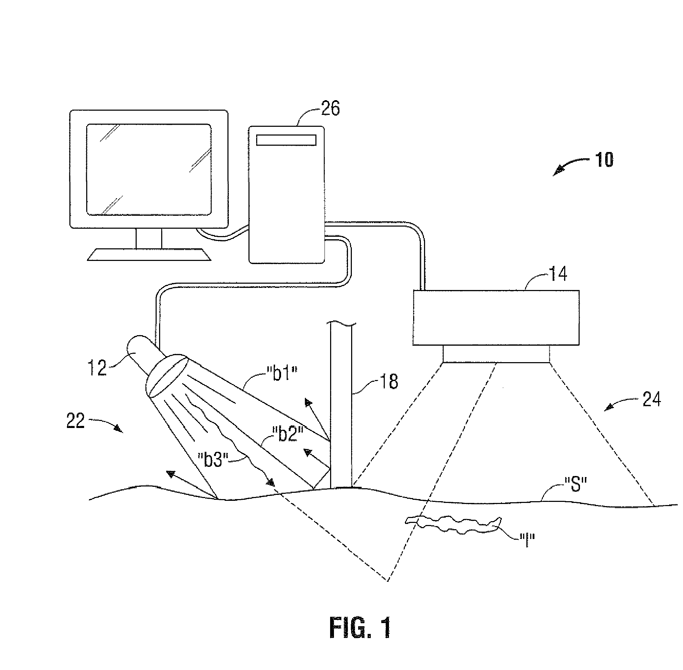

Referring initially to FIG. 1, an embodiment of an apparatus 10 is depicted for employing light to diagnose a suspected lesion "l" in a patient's skin "s." The apparatus includes a light source 12, a detector 14 and a light barrier 18 disposed therebetween. The light source 12 emits light toward an illumination region 22 on a first side of the light barrier 18, and the detector 14 receives light from an examination region 24 on a second side of the light barrier. The light barrier 18 is substantially impervious to the light emitted from the light source 12, and intimately engages the surface of the skin "s" to define the boundary between the illumination region 22 and the examination region 24.

The light barrier 18 is substantially impervious to the light directed toward the illumination region 22 such that only light passing beneath the light barrier 18 and through the skin "s" passes from the illumination region 22 into the examination region 24. A first beam of light "bl" is reflected by the light barrier 18, and is not permitted to enter the examination region 24. The light barrier 18 may also be configured to absorb, rather than reflect, light contacting its surface in the illumination region 22. A second beam of light "b2" encounters the light barrier 18 after reflection from the surface of the skin "s" in the illumination region 22. The second beam of light "b2" is also reflected from the surface of the light barrier 18 irrespective of any characteristic changes induced by reflecting off the skin "S." A third beam of light "b3" penetrates the surface of the skin "S" in the illumination region 22 and is reflected into the examination region 24 underneath the light barrier 18. In this manner, the light barrier 18 permits only light penetrating the skin "S" to enter the examination region 24 and to encounter the detector 14.

The light source 12 may be configured to emit broadband light, light at specific frequencies or wavelengths, and/or light within specific frequency and wavelength bands. One suitable light source is a quartz tungsten halogen (QTH) lamp. This type of lamp produces a stable output and smooth spectral curve in the visible and infrared frequency bands. Radiation with longer wavelengths tends to penetrate more deeply into the skin. Thus, a light source 12 having an appropriate wavelength for a suspected lesion "l " at a known depth may be selected, or the light source 12 may be configured to permit an operator to selectively vary the wavelength light emitted. Filters may be provided with the light source 12 to prevent unwanted wavelengths from entering the illumination region 22. Other appropriate devices for the light source 12 include incandescent or fluorescent lamps, LEDs, He-Cd lasers and natural sunlight.

The detector 14 may include various devices to monitor the light emanating from the examination region 24. For example, the detector 14 may include an array of sensors sensitive to particular wavelengths of light. Also, an optical signal receiver such as a CCD camera may be employed to produce a photographic image of the examination region 24. The CCD camera may be configured to convert optical brightness or intensity of light in the visible spectrum into electrical amplitude signals, and digitize an image of the examination region 24. The digitized image and amplitude signals may then be transmitted to a processor such as an image processing board included in a personal computer 26. Where spectroscopic resolution of the examination region 24 is to be analyzed, the detector 14 may be adapted to acquire a spectrally-resolved image of the examination region 24. A device such as an imaging spectrometer may be employed as the detector 14 to provide spatial and spectroscopic resolution. For example, an imaging spectrometer may be configured to receive and distinguish intensities of light at various wavelengths (or frequencies) or ranges of wavelengths (or ranges of frequncise) both within and beyond the visible spectrum. Frequency dependent data generated by the imaging spectrometer may then be transmitted to the personal computer 26 for analysis.

The computer 26 is coupled to both the light source 12 and the detector 14. Thus, the computer 26 may serve as a controller to initiate and vary the illumination of the illumination region 22, and also to coordinate the detection of light from the examination region 24. The image processing board of the computer 26 may be equipped with suitable software or instructions for analyzing the spatial and/or spectral distribution of light from the examination region. For example, mathematical algorithms that distinguish malignant lesions from benign lesions may be employed by the image processing board. The computer 26 may also be equipped with a memory for data storage. Libraries of data describing the characteristics or signatures of known benign and malignant lesions may be preloaded into the memory of the computer 26. Alternatively or additionally, the memory may be preloaded with previously recorded images of the suspected lesion "l " and the surrounding skin "s" for comparison with newly recorded images.

In use, the apparatus 10 is placed such that the suspected lesion "l" is located generally within examination region 24. Alternate placements of the apparatus 10 with respect to the suspected lesion "l " may also be effective. For example, apparatus 10 may be placed such that the suspected lesion "l " lies directly beneath the light barrier 18, or generally within the illumination region 22. Once the apparatus is in position, the light source 12 is activated to illuminate the surface of the skin "s" within the illumination region 22. The entire surface may be illuminated at once, or a small spot scanning method may be employed. The light passing though the skin "s" into the examination region 24 is collected by the detector 14, and data indicative of the collected light is generated. The data is transmitted to the computer 26 for analysis and/or comparison with an appropriate reference. The computer 26 may then instruct the light source 12 and detector 14 to sequentially repeat the process with light having differing characteristics until the boundaries of the suspected lesion "l " may be ascertained and an appropriate diagnosis may be made.

In some instances, upon reviewing the analysis preformed by the computer 26, a clinician may decide to remove the lesion "l." The computer 26 may also be coupled to an automated removal apparatus (not shown) such that information regarding the exact boundaries of the lesion "l" may be transmitted to the automated removal apparatus. The removal apparatus may then employ light energy or mechanically manipulate the skin "s" in only those areas necessary to remove the lesion "l."

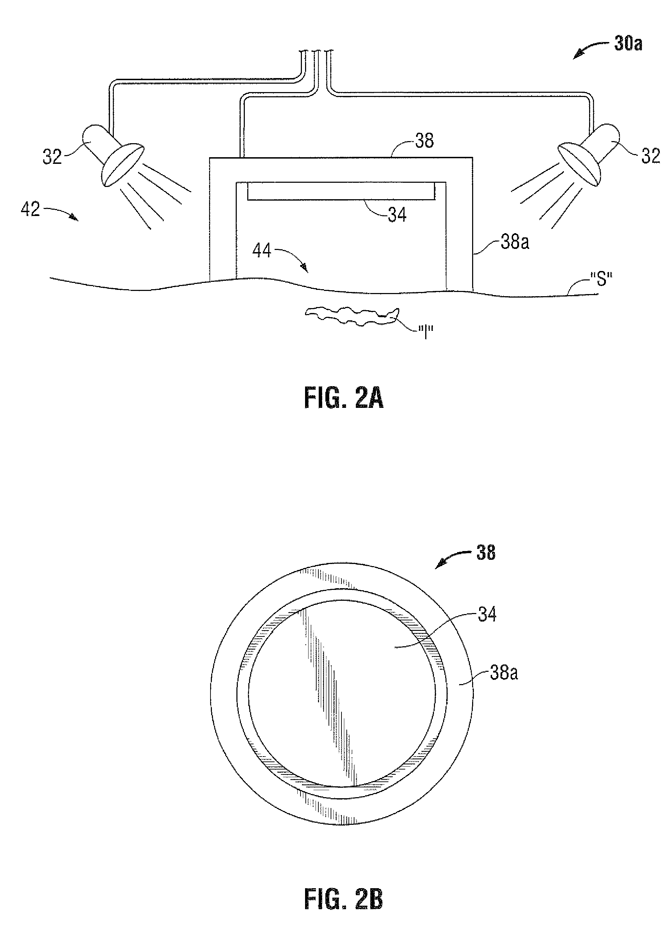

Referring now to Figures 2A and 2B, an alternate embodiment of a diagnostic apparatus 30a is depicted for assessing a suspected lesion "l." The apparatus 30a includes a plurality of light sources 32, a detector 34 and a light barrier 38. The light sources 32 and detector 34 are configured for connection to a computer 26, as in the embodiment described above with reference to Figure 1. The light barrier 38 includes an annular wall 38a extending from the detector 34 to the surface of the skin "s." In this way, the light barrier 38 defines an exterior illumination region 42 and an interior examination region 44. The interior examination region 44 is fully contained to discourage ambient light from entering the examination region 44 and altering the distribution of light encountering the detector 34. The light barrier 38 may be positioned such that the annular wall 38a substantially encircles the lesion "l," and the plurality of light sources 32 may be radially positioned to surround the light barrier 38. In this manner, the illumination region 42 may be evenly illuminated in the vicinity of the annular wall 38a, and the examination region 44 may be evenly illuminated only by light passing through the skin "s."

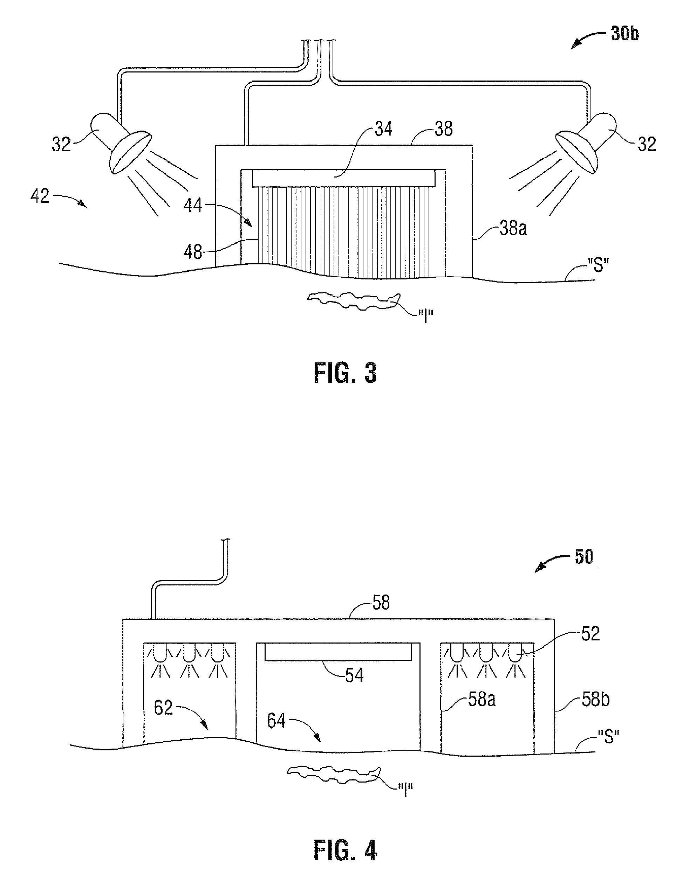

Figure 3 depicts an alternate diagnostic apparatus 30b. The apparatus 30b is substantially similar to the apparatus 30a described above with the addition of a light transmission structure 48. The light transmission structure 48 extends between the surface of the skin "s" in the examination region 44 and the detector 34.

The light transmission structure 48 may comprise a fiber-optic bundle or a light pipe constructed from an effective light-transmissive material, such as plastic or glass, to carry the light emanating from the skin "s" in the examination region 44 to the detector 34. By providing a light transmission structure 48 with individual fibers spread over the examination region 44, light may be transmitted from each area of the examination region 44 to the detector 34 in a known or predetermined direction. In this manner, light scattering may be discouraged and an accurate spatial distribution at which light emanates from the surface of the skin "S" may be ascertained.

In other embodiments (not shown) the light transmission structure 48 may extend beyond the light barrier 38 to transmit light from the examination region 44 to a detector 34 that is disposed remotely with respect to the light barrier 38. In still other embodiments (not shown) a light transmission structure may be provided to guide light from the light source 32 to the surface of the skin "s" in the illumination region 42.

Referring now to Figure 4, another alternate embodiment of a diagnostic apparatus 50 is depicted. The apparatus 50 includes a plurality of light sources 52, a detector 54 and a light barrier 58. The light barrier 58 includes a pair of annular walls 58a, 58b extending to the surface of the skin "s." A first annular wall 58a protrudes from a radial position between the light sources 52 and the detector 54, and the second annular wall protrudes from a radial position outside the light sources 52. In this manner, the annular walls 58a, 58b define an interior illumination region 62 that substantially surrounds a central interior examination region 64.

The second annular wall 58b serves to separate the illumination region 62 from the ambient environment surrounding the light barrier 58. Ambient light is discouraged from entering the illumination and examination regions 62, 64, and also, light emitted from the light sources 52 is discouraged from entering the ambient environment. Thus, the second annular wall 58b may serve to protect the patient and clinicians in the ambient environment from dangerous light emissions such as ultraviolet or laser light when light sources 52 are so configured.

The light sources 52 and detector 54 are both mechanically and electrically coupled to the light barrier 58 of diagnostic apparatus 50. Thus, the light barrier 58 may be coupled to a computer 26 as described above with reference to Figure 1 to provide control and analysis functionality. The mechanical coupling of the light sources 52 and detector 54 to the light barrier 58 maintains a spatial relationship between the light sources 52 and detector 54, and thus the light barrier 58 provides a means for consistent application of light to the examination region 64.

Although the foregoing disclosure has been described in some detail by way of illustration and example, for purposes of clarity or understanding, it will be obvious that certain changes and modifications may be practiced within the scope of the appended claims.

## Claims

1. An apparatus (10) for examining a region of skin, the apparatus comprising:
a light source (12) for emitting light toward an illumination region (22) of a skin surface;
a detector (14) for receiving light from an examination region (24) of the skin surface; and
a light barrier (18) for intimately contacting the skin surface to define a boundary between the illumination region and the examination region, the light barrier substantially impervious to the light emitted toward the illumination region.

2. The apparatus according to claim 1, wherein the light source is configured to emit light in the visible and near infrared frequency bands.

3. The apparatus according to claim 1 or 2, wherein the light source is configured to vary the frequency of the emitted light.

4. The apparatus according to claim 1, 2 or 3, wherein the detector is configured to acquire a spectrally-resolved image of the examination region and to provide measurements of the light (e.g. light intensity) as a function of at least one of frequency and wavelength.

5. The apparatus according to claim 1, 2, 3 or 4, wherein the light barrier includes a first annular wall (38a, 58a) substantially encircling the examination region.

6. The apparatus according to claim 5, wherein the illumination region (42, 62) substantially encircles the first annular wall, and wherein the light source is configured to emit light substantially evenly about the illumination region.

7. The apparatus according to claim 6, wherein the light barrier includes a second annular wall (58b) substantially encircling the illumination region (62).

8. The apparatus according to any one of the preceding claims, wherein the light source and the detector are fixedly coupled to the light barrier such that a spatial relationship is maintained therebetween.

9. The apparatus according to any one of the preceding claims, wherein the detector includes a camera.

10. The apparatus according to claim 9, wherein the camera is configured to digitize an image of the examination region and transmit the digitized image to a processor (26) for analysis.

11. The apparatus according to claim 10, wherein the processor comprises a personal computer.

12. The apparatus according to claim 11, wherein the personal computer includes at least one reference image for comparison with the digitized image of the examination region.

13. The apparatus according to any one of the preceding claims, wherein the detector is optically coupled to a light transmission structure extending in the direction of the examination region, the light transmission structure configured to guide light to the detector.

14. The apparatus according to claim 13, wherein the light transmission structure includes at least one of the group consisting of a light pipe and a fiber-optic bundle.
